# EUROPÄISCHE PATENTSCHRIFT

(11) **EP 2 899 276 B1**
(45) Veröffentlichungstag und Bekanntmachung des Hinweises auf die Patenterteilung: **03.10.2018**
(21) Anmeldenummer: 15152388.3
(22) Anmeldetag: 25.05.2009
(51) Int. Cl.: C12N 1/06, C12N 15/10

(54) **Lyse-, Binde- und/oder Waschreagenz verwendbar zur Isolierung und/oder Reinigung von Nukleinsäuren**
Lysis, binding and/or washing reagent which can be used for isolating and/or cleaning nucleic acids
Réactif de nettoyage et/ou de liaison ou de lyse utilisable pour l'isolation et/ou le nettoyage d'acides nucléiques

(30) Priorität: 30.05.2008 DE 102008026058
(43) Veröffentlichungstag der Anmeldung: 29.07.2015
(62) Teilanmeldung aus: 09753837.5
(73) Patentinhaber: QIAGEN GmbH, 40724 Hilden (DE)
(72) Erfinder: Fabis, Roland, 51375 Leverkusen (DE); Homann-Wischinski, Anke, 40724 Hilden (DE); Voss, Thorsten, 40724 Hilden (DE); Hanselle, Thomas, 40724 Hilden (DE)
(74) Vertreter: Roth, Carla

(56) Entgegenhaltungen:
- WO-A-00/77235
- WO-A-2006/023471
- WO-A-2006/073497
- WO-A-2007/060248
- DE-A1- 10 147 439
- CULL M ET AL: "Preparation of extracts from prokaryotes", METHODS IN ENZYMOLOGY; [METHODS IN ENZYMOLOGY], ACADEMIC PRESS INC, SAN DIEGO, CA, US, Bd. 182, 1. Januar 1990 (1990-01-01), Seiten 147-153, XP008106338,
- JOHANSSON F ET AL: "Brij 58, a polyoxyethylene acyl ether, creates membrane vesicles of uniform sidedness. A new tool to obtain inside-out (cytoplasmic side-out) plasma membrane vesicles.", THE PLANT JOURNAL : FOR CELL AND MOLECULAR BIOLOGY JAN 1995, Bd. 7, Nr. 1, Januar 1995 (1995-01), Seiten 165-173, XP002542853, ISSN: 0960-7412
- MULLER D E ET AL: "Maturation of parvovirus LuIII in a subcellular system. I. Optimal conditions for in vitro synthesis and encapsidation of viral DNA.", THE JOURNAL OF GENERAL VIROLOGY MAY 1983, Bd. 64, Nr. Pt 5, Mai 1983 (1983-05), Seiten 1043-1054, XP002542854, ISSN: 0022-1317
- KELLERMAYER M ET AL: "Release of potassium, lipids, and proteins from nonionic detergent treated chicken red blood cells", JOURNAL OF CELLULAR PHYSIOLOGY, WILEY LISS, NEW YORK, NY, US, Bd. 159, Nr. 2, 1. Mai 1994 (1994-05-01), Seiten 197-204, XP008110703, ISSN: 0021-9541
- Sigma: "Life Detergents and Solubilization Reagents Proteomics Kits and Reagents Detergent Properties and Applications BioUltra Detergents Cyclodextrins Antifoams", , 1 January 2008 (2008-01-01), XP055368927, Retrieved from the Internet: URL:http://www.sigmaaldrich.com/content/da m/sigma-aldrich/docs/Sigma/General_Informa tion/2/biofiles_issue3_3.pdf [retrieved on 2017-05-03]
- Embl: "Protein Purification - Extraction and Clarification - Solubilisation of membrane proteins - EMBL", , 1 January 2002 (2002-01-01), XP055368929, Retrieved from the Internet: URL:https://www.embl.de/pepcore/pepcore_se rvices/protein_purification/extraction_cla rification/solubilisation_membrane_protein s/ [retrieved on 2017-05-03]

## Beschreibung

### Gebiet der Erfindung

Die vorliegende Erfindung betrifft ein Lyse-, Binde- und/oder Waschreagenz sowie ein Verfahren zur Isolierung und/oder Reinigung von Nukleinsäuren. Das Lyse-, Binde- und/oder Waschreagenz sowie das Verfahren sind insbesondere für Anwendungszwecke in der molekularen Diagnostik geeignet.

### Technischer Hintergrund

Im Stand der Technik sind eine Vielzahl von Verfahren zur Isolierung und/oder Reinigung von Nukleinsäuren wie Desoxyribonukleinsäure (DNA) oder Ribonukleinsäure (RNA) aus Zellen, Zellkulturen oder Viruskulturen bekannt.

Hierbei beruhen "klassische" Verfahren zur Isolierung von Nukleinsäuren, die vielfach manuell durchgeführt werden, auf einem einstufigen Verfahren, bei dem nach Zusatz eines wässrigen Puffers und eines organischen Extraktionsmittels eine Extraktion durchgeführt wird. Die Nukleinsäuren verbleiben in der wässrigen Phase und können nach Abtrennung der organischen Phase, die unerwünschte Begleitstoffe enthält, isoliert werden.

Diese Verfahren verwenden zum Einen üblicherweise gesundheitsschädigende organische Extraktionsmittel wie Chloroform oder Phenol, zum anderen verbleiben wasserlösliche Verunreinigungen in der die Nukleinsäuren enthaltenden wässrigen Phase, die in weiteren Reinigungsschritten abgetrennt werden müssen.

Daher hat im Stand der Technik ein alternatives Verfahren an Bedeutung gewonnen, das auf der selektiven Adsorption von Nukleinsäuren an festen, meist mineralischen Trägern wie Siliciumdioxid, basiert. Das Bindeprinzip basiert auf einer reversiblen Bindung der Nukleinsäuren unter Einfluss von so genannten chaotropen Salzen und/oder Alkohol an die Siliciumdioxidoberfläche. In einem mehrstufigen Verfahren werden der Nukleinsäuren-enthaltenden Probe verschiedene Lösungen oder Mischungen, meist Lyse-, Binde-, Wasch- und/oder Elutionslösungen oder -mischungen, zugesetzt und in einem abschließenden Verfahrensschritt wird die gereinigte Nukleinsäure von dem spätestens im Bindeschritt zugegebenen Träger eluiert.

Das Grundprinzip beider Verfahren beruht darauf, dass in einem ersten Schritt die Zellen, insbesondere die pflanzlichen, tierischen, menschlichen, bakteriellen oder Virus-Zellen lysiert werden. Hierzu werden die Zellen zunächst mit einem Lysepuffer inkubiert, der die Zellen aufschließt.

Im Stand der Technik sind Puffer und Verfahren zum Lysieren zellulärer Materialien einer biologischen Probe bekannt. Bekannte Lysepuffer enthalten häufig das Tensid Polyoxyethylensorbitanmonolaurat (Tween® 20). Dieses Tensid wird dazu verwendet, im Rahmen der Zelllyse Verunreinigungen in einen löslichen oder stabilisierten Zustand zu überführen, um diese von der Nukleinsäure abzutrennen.

Nachteilig an Polyoxyethylensorbitanmonolaurat (Tween® 20) enthaltenden Lysepuffern ist, dass diese bei Lagerung nicht stabil sind. So sinkt beispielsweise der pH Wert ab. Nachteilig ist insbesondere, dass bei der Verwendung dieser Lysepuffer nach Lagerung die Ausbeute der isolierten Nukleinsäuren abnimmt. Darüber hinaus ist nachteilig, dass die Nukleinsäuren enthaltenden Eluate getrübt sind, was auf die Anwesenheit von Verunreinigungen hinweist, die die weitere Verwendung der isolierten Nukleinsäuren stören können.

WO 2006/023471 A2 offenbart einen Lysepuffer umfassend eine chaotrope Verbindung, ein Detergenz und einen Puffer.

WO 2006/073497 A1 offenbart ein Lysereagenz mit stark alkalischem pH-Wert.

Cull et al. (Methods in Enzymology, 1990, Bd. 182, Seiten 147-153) beschreiben Lyseverfahren zur Herstellung von Extrakten aus Prokaryoten.

Johansson et al. (The Plant Journal, 1995, Bd. 7, Seiten 165-173) beschreiben die Verwendung von Brij® 58 zur Herstellung von Plasmamembranvesikeln.

Muller et al. (Journal of General Virology, 1983, Bd. 64, Seiten 1043-1054) beschreiben die Reifung von Parvovirus LuIII in einem subzellulären System sowie die Herstellung von mit Brij® 58 lysierten Zellen.

Kellermayer et al. (Journal of Cellular Physiology, 1994, Bd. 159, Seiten 197-204) beschreiben die Freisetzung von Kalium, Lipiden und Proteinen aus mit nichtionischem Detergenz behandelten roten Blutzellen.

DE 101 47 439 A1 betrifft ein Verfahren zur Isolierung von DNA aus biologischen Proben, insbesondere aus humanem Vollblut, bei dem ein Detergenzienhaltiges Lysereagenz eingesetzt wird.

Sigma (BioFiles Life Science Research, 2008, Bd. 3(3) "Detergents and Solubilization Reagents") und EMBL (2002, "Protein Purification - Extraction and Clarification - Solubilization of Membrane Proteins") offenbaren verschiedene Detergenzien.

Aufgabe der vorliegenden Erfindung war es daher, ein Mittel zur Verfügung zu stellen, das wenigstens einen der vorgenannten Nachteile des Standes der Technik überwindet und dabei möglichst gleich gute oder bessere Lyse-, Binde- und/oder Wascheigenschaften aufweist.

Die Aufgabe wird durch ein Lyse-, Binde- und/oder Waschreagenz gemäß Anspruch 1 der vorliegenden Erfindung gelöst. Demgemäß wird ein Lyse-, Binde- und/oder Waschreagenz zur Verfügung gestellt umfassend:
- wenigstens eine chaotrope Verbindung,
- wenigstens eine Pufferverbindung vorzugsweise ausgewählt aus der Gruppe umfassend Tris(hydroxymethyl)aminomethan, N-(Tri(hydroxymethyl)methyl)glycin, N,N-Bis(2-hydroxyethyl)-glycin, 3-(N-Morpholino)propansulfonsäure, N-(2-Hydroxyethyl)piperazin-N'-(2-ethansulfonsäure), Piperazin-1,4-bis(2-ethansulfonsäure), N-Cyclohexyl-2-aminoethansulfonsäure, 2-(N-Morpholino)-ethansulfonsäure und/oder Phosphatpuffer, und
- wenigstens ein polyoxyethylen-basiertes nicht-ionisches Tensid ausgewählt aus der Gruppe bestehend aus Polyoxyethylennonylphenylether, Polyoxyethylen(5)isooctylphenylether und Polyoxyethylen(12)isooctylphenylether im Bereich von ≥ 8 % Gewicht/Volumen bis ≤ 50 % Gewicht/Volumen, bezogen auf das Gesamtvolumen des Reagenzes.

Offenbart wird ein Lyse-, Binde- und/oder Waschreagenz umfassend:
- wenigstens eine chaotrope Verbindung,
- wenigstens eine Pufferverbindung vorzugsweise ausgewählt aus der Gruppe umfassend Tris(hydroxymethyl)aminomethan (TRIS), N-(Tri(hydroxymethyl)methyl)glycin (Tricin), N,N-Bis(2-hydroxyethyl)-glycin (BICIN), N-(2-Hydroxyethyl)piperazin-N'-(2-ethansulfonsäure) (HEPES), Piperazin-1,4-bis(2-ethansulfonsäure) (PIPES), N-Cyclohexyl-2-aminoethansulfonsäure (CHES), 2-(N-Morpholino)-ethansulfonsäure (MES), 3-(N-Morpholino)propansulfonsäure (MOPS) und/oder Phosphatpuffer, und
- wenigstens ein polyoxyethylen-basiertes nicht-ionisches Tensid ausgewählt aus der Gruppe umfassend Polyoxyethylen-Fettalkoholether, Polyoxyethylen-Alkylphenylether und/oder Polyoxyethylen-Polyoxypropylen Blockcopolymere im Bereich von ≥ 8 % (Gewicht/Volumen) bis ≤ 50 % (Gewicht/Volumen), bezogen auf das Gesamtvolumen des Reagenzes.

Unter dem Begriff "Lyse-, Binde- und/oder Waschreagenz" werden im Sinne der vorliegenden Erfindung Reagenzien verstanden, die Lysereagenzien, Bindereagenzien oder Waschreagenzien sind, wie auch Reagenzien, die sowohl als Lysewie auch als Binde- als auch als Waschreagenz wirken können. Insbesondere werden im Sinne der vorliegenden Erfindung unter dem Begriff "Lyse-, Binde- und/oder Waschreagenz" auch Mischungen aus erfindungsgemäßen Lysereagenzien, Bindereagenzien und/oder Waschreagenzien verstanden.

Unter dem Begriff "Reagenz" werden im Sinne der vorliegenden Erfindung Lyse-, Binde- und/oder Waschreagenz verstanden.

Unter dem Begriff "chaotrope Verbindung" im Sinne der vorliegenden Erfindung werden Verbindung verstanden, die denaturierend auf Proteine wirken und die insbesondere die regelmäßige, auf Bildung von Wasserstoffbrückenbindungen beruhende Struktur, von flüssigem Wasser zerstören.

Unter dem Begriff "Pufferverbindung" im Sinne der vorliegenden Erfindung werden Verbindungen verstanden, die eine Pufferung oder Stabilisierung des pH-Werts einer wässrigen Lösung zur Verfügung stellen können.

Unter dem Begriff "Phosphatpuffer" werden im Sinne der vorliegenden Erfindung Phosphatsalze wie Dihydrogenphosphate, beispielsweise Kaliumdihydrogenphosphat (KH₂PO₄) oder Natriumdihydrogenphosphat (NaH₂PO₄) und Hydrogenphosphate, beispielsweise Dinatriumhydrogenphosphat-Dihydrat (Na₂HPO₄ · 2 H₂O) oder Dikaliumhydrogenphosphat verstanden. Die Verwendung von Mischungen der Phosphatsalze ist ebenfalls möglich. Ein weiterer gängiger Phosphatpuffer ist PBS (phosphate buffered saline), der Natriumchlorid, Na₂HPO₄, Kaliumchlorid und KH₂PO₄ enthält.

Unter dem Begriff "Nukleinsäure" im Sinne der vorliegenden Erfindung werden insbesondere - aber nicht darauf beschränkt - natürliche, vorzugsweise isolierte lineare, verzweigte oder zirkuläre Nukleinsäuren wie RNA, insbesondere mRNA, siRNA, miRNA, snRNA, tRNA, hnRNA oder Ribozyme, DNA, Plasmid DNA und dergleichen, synthetische oder modifizierte Nukleinsäuren, in vitro Transkripte, beispielsweise Oligonukleotide, insbesondere für die PCR verwendbare Primer, Sonden oder Standards, mit Digoxigenin, Biotin oder Fluoreszensfarbstoffen markierte Nukleinsäuren, methylierte Nukleinsäuren oder sogenannte PNAs ("peptide nucleic acids") verstanden.

Unter dem Begriff "Tensid" werden im Sinne der vorliegenden Erfindung grenzflächenaktive und/oder oberflächenaktive Substanzen verstanden.

Unter dem Begriff "Fettalkohol" werden im Sinne der vorliegenden Erfindung Alkohole mit einer Kettenlänge einer Anzahl von sechs bis 22 Kohlenstoffatomen bevorzugt 8 bis 20 Kohlenstoffatomen, vorzugsweise 10 bis 18 Kohlenstoffatomen, besonders bevorzugt 12 bis 18 Kohlenstoffatomen verstanden. Insbesondere bevorzugt sind Alkohole mit einer Anzahl von 12, 14, 16 oder 18 Kohlenstoffatomen. Die Fettalkohole können zwar einfach oder mehrfach ungesättigt sein, bevorzugt handelt es sich jedoch um gesättigte Fettalkohole.

"Polyoxyethylen" steht im Sinne der vorliegenden Erfindung für eine HO-(CH₂CH₂O)ₙ-Einheit, wobei n vorzugsweise eine ganze Zahl von 2 bis 150, weiter bevorzugt von 4 bis 120, noch weiter bevorzugt von 8 bis 80 und am meisten bevorzugt eine ganze Zahl ausgewählt aus 2, 3, 4, 5, 6, 7, 8, 9,10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149 oder 150, darstellt.

"Polyoxypropylen" steht im Sinne der vorliegenden Erfindung für eine HO-(CH₂CH₂CH₂O)ₙ-Einheit, wobei n vorzugsweise eine ganze Zahl von 10 bis 90, weiter bevorzugt von 20 bis 80, noch weiter bevorzugt von 30 bis 70 ist und am meisten bevorzugt ist n eine ganze Zahl ausgewählt aus 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89 oder 90.

Unter der Angabe "% Gewicht/Volumen", "% (Gewicht/Volumen)" oder "% (w/v)" ist im Sinne der vorliegenden Erfindung beispielsweise die Grammangabe des Tensids pro 100 ml Reagenz oder Zusammensetzung zu verstehen.

In überraschender Weise wurde gefunden, dass die erfindungsgemäßen Lyse-, Binde- und/oder Waschreagenzien eine verbesserte Stabilität bei Lagerung aufweisen. So können erfindungsgemäße Lyse-, Binde- und/oder Waschreagenzien beispielsweise während einer Lagerung bei Raumtemperatur von drei, vorzugsweise sechs Monaten, weiter bevorzugt mindestens acht Monaten einen stabilen pH-Wert aufweisen. Insbesondere können erfindungsgemäße Lyse-, Binde- und/oder Waschreagenzien auch bei einer Lagerung bei erhöhten Temperaturen beispielsweise bei 50°C über mehrere Wochen hinweg, vorzugsweise über mehrere Monate, einen stabilen pH-Wert aufweisen.

Dies hat sich für Lyse-, Binde- und/oder Waschreagenzien als vorteilhaft herausgestellt, da vermutet wird, dass die Instabilität des pH-Wertes mit dem Auftreten von Verunreinigungen in dem nach der Isolation erhaltenen Eluat enthaltend die Nukleinsäuren in Verbindung steht.

Polyoxyethylen-basierte nicht-ionische Tenside haben sich für eine breite Spanne von Anwendungen innerhalb der vorliegenden Erfindung als vorteilhaft herausgestellt. Insbesondere bei Lyse-, Binde- und/oder Waschreagenzien umfassend Pufferverbindungen vorzugsweise ausgewählt aus der Gruppe umfassend Tris(hydroxymethyl)aminomethan (TRIS), N-(Tri(hydroxymethyl)methyl)glycin (Tricin), N,N-Bis(2-hydroxyethyl)-glycin (BICIN), N-(2-Hydroxyethyl)piperazin-N'-(2-ethansulfonsäure) (HEPES), Piperazin-1,4-bis(2-ethansulfonsäure) (PIPES), N-Cyclohexyl-2-aminoethansulfonsäure (CHES), 2-(N-Morpholino)-ethansulfonsäure (MES), 3-(N-Morpholino)propansulfonsäure (MOPS) und/oder Phosphatpuffer kann eine verbesserte Stabilität bei Lagerung beobachtet werden.

Es konnte festgestellt werden, dass vorteilhafte Effekte der erfindungsgemäßen Lyse-, Binde- und/oder Waschreagenzien insbesondere bei einem Gehalt an polyoxyethylen-basiertem nicht-ionischem Tensid, Polyoxyethylen-Alkylphenylether, im Bereich von ≥ 8 % (Gewicht/Volumen) bis ≤ 50 % (Gewicht/Volumen), bezogen auf das Gesamtvolumen des Lyse-, Binde- und/oder Waschreagenzes, auftraten.

Für den Fall, dass Mischungen von Tensiden verwendet werden, handelt es sich bei den Konzentrationsangaben vorzugsweise um den Gesamtgehalt an Tensid, beispielsweise im Bereich von insgesamt ≥ 8 % (Gewicht/Volumen) bis ≤ 50 % (Gewicht/Volumen), bezogen auf das Gesamtvolumen des Reagenzes.

Dies hat sich insbesondere für Lysereagenzien innerhalb der vorliegenden Erfindung als vorteilhaft herausgestellt.

Ein weiterer Vorteil des erfindungsgemäßen Lyse-, Binde- und/oder Waschreagenzes kann dadurch zur Verfügung gestellt werden, dass erfindungsgemäße Lyse-, Binde- und/oder Waschreagenzien bei einer Verwendung zur Isolierung und/oder Reinigung von Nukleinsäuren auch nach einer mehrwöchigen oder mehrmonatigen Lagerung des Lyse-, Binde- und/oder Waschreagenzes bei Raumtemperatur oder bei erhöhten Temperaturen beispielsweise bis 50°C eine unverändert gute Ausbeute der isolierten Nukleinsäuren zeigen, während Puffer aus dem Stand der Technik, insbesondere Puffer, die Tween®-20 enthalten, nach Lagerung niedrigere Ausbeuten an Nukleinsäuren zeigen.

Insbesondere ist von Vorteil, dass bei Verwendung erfindungsgemäßer Lyse-, Binde- und/oder Waschreagenzien auch nach mehrwöchiger oder mehrmonatiger Lagerung ein Eluat enthaltend die Nukleinsäuren nicht oder nur geringfügig getrübt ist. Ein Vorteil ist somit, dass keine oder zumindest deutlich weniger Verunreinigungen im Eluat enthalten sein können, wodurch die weitere Verwendung des die Nukleinsäuren enthaltenden Eluats wesentlich vorteilhafter ist, da weitere zeitaufwendige und die Ausbeute an Nukleinsäuren verringernde Reinigungsschritte entfallen können.

Offenbart wird, dass insbesondere Lyse-, Binde- und/oder Waschreagenzien umfassend Polyoxyethylen-Fettalkoholether insbesondere Polyoxyethylen-Cetyl- oder Oleylalkoholether sich im Vergleich durch eine besonders gute Ausbeute an isolierten Nukleinsäuren insbesondere an Virus-DNA auszeichnen. Insbesondere konnte überraschend festgestellt werden, dass eine Isolation der DNA von Hepatitis-B-Virus (HBV) im Vergleich zu Tween® 20-haltigen Lysepuffern sowohl bei Verwendung eines frisch angesetzten Lyse- und/oder Bindereagenzes enthaltend Polyoxyethylen-Cetylalkoholether wie auch bei Verwendung nach mehreren Wochen, insbesondere mehrere Monate Lagerung bei 50°C eine deutlich erhöhte Ausbeute an Virus-DNA erhalten werden konnte. Dies kann insbesondere einen besonderen Vorteil des Lyse- und/oder Bindereagenzes zur Verfügung stellen, da das Hepatitis-B-Virus (HBV) als schwer lysierbares Virus gilt. Das erfindungsgemäße Lysereagenz ist insbesondere geeignet zur Isolation von Virus-DNA.

Das erfindungsgemäße Lyse-, Binde und/oder Waschreagenz umfasst wenigstens einen Polyoxyethylenalkylphenylether als polyoxyethylen-basiertes nicht-ionisches Tensid. Das beanspruchte Lyse-, Binde und/oder Waschreagenz umfasst wenigstens ein polyoxyethylen-basiertes nicht-ionisches Tensid ausgewählt aus der Gruppe bestehend aus Polyoxyethylennonylphenylether, Polyoxyethylen(5)isooctylphenylether und Polyoxyethylen(12)isooctylphenylether im Bereich von ≥ 8 % Gewicht/Volumen bis ≤ 50 % Gewicht/Volumen, bezogen auf das Gesamtvolumen des Reagenzes.

Offenbart wird, dass Polyoxyethylenalkylphenylether eine Alkylgruppe mit fünf bis 15 Kohlenstoffatomen, oder mit 6 bis 10 Kohlenstoffatomen, aufweisen können. Weiter offenbart sind verzweigte oder unverzweigte C₇- bis C₁₀-Alkylgruppen, insbesondere verzweigte oder unverzweigte C₈- und C₉-Alkylgruppen, besonders Isooctyl- Gruppen und Nonyl-Gruppen.

Polyoxyethylennonylphenylether und Polyoxyethylenisooctylphenylether sind beispielsweise erhältlich unter der Handelsbezeichnung Igepal®, beispielsweise von der Firma BASF.

Beispiele geeigneter Polyoxyethylennonylphenylether und Polyoxyethylenisooctylphenylether sind vorzugsweise ausgewählt aus der Gruppe umfassend Polyoxyethylen(2)nonylphenylether (Igepal® CO-210), Polyoxyethylen(5)nonylphenylether (Igepal® CO-520), Polyoxyethylen(5)isooctylphenylether (Igepal® CA-520), Polyoxyethylen(9)nonylphenylether (Igepal® CO-630), Polyoxyethylen(12)nonylphenylether (Igepal® CO-720), Polyoxyethylen(12)isooctylphenylether (Igepal® CA-720) und/oder Polyoxyethylen(100)nonylphenylether (Igepal® CO-990).

Gemäß einer bevorzugten Ausführungsform der Erfindung umfasst das Lyse-, Binde- und/oder Waschreagenz nicht-ionisches Tensid im Bereich von ≥ 9 % (Gewicht/Volumen) bis ≤ 40 % (Gewicht/Volumen), vorzugsweise im Bereich von ≥ 10 % (Gewicht/Volumen) bis ≤ 30 % (Gewicht/Volumen), bevorzugt im Bereich von ≥ 15 % (Gewicht/Volumen) bis ≤ 20 % (Gewicht/Volumen), bezogen auf das Gesamtvolumen des Reagenzes.

Gemäß einer bevorzugten Ausführungsform der Erfindung ist die chaotrope Verbindung ein Natrium- oder Guanidiniumsalz, vorzugsweise ausgewählt aus der Gruppe umfassend Natriumiodid, Natriumperchlorat, Guanidiniumhydrochlorid, Guanidiniumthiocyanat, Guanidiniumisothiocyanat und/oder eine Mischung zweier oder mehrerer Salze davon. Bevorzugt ist die chaotrope Verbindung ein Guanidiniumsalz, vorzugsweise ausgewählt aus der Gruppe umfassend Guanidiniumhydrochlorid, Guanidiniumthiocyanat und/oder Guanidiniumisothiocyanat.

Insbesondere hat sich eine Kombination der vorgenannten chaotropen Verbindungen und der erfindungsgemäß enthaltenen polyoxyethylen-basierten nicht-ionischen Tenside für die Lyse von Viruszellen und die Isolierung von Nukleinsäuren aus Viruszellen als günstig erwiesen.

Geeignete Konzentrationen und Mengen der chaotropen Verbindungen können in Abhängigkeit von der Art der Proben oder den Parametern der Lyse variieren, wobei Konzentrationen der chaotropen Verbindung im Bereich von ≥ 0,1 M bis ≤ 10 M generell günstig sind, bezogen auf das Gesamtvolumen des Reagenzes. Vorzugsweise liegt die Konzentrationen der chaotropen Verbindung des Lyse-, Binde- und/oder Waschreagenzes im Bereich von ≥ 0,5 M bis ≤ 8 M, bevorzugt im Bereich von ≥ 0,9 M bis ≤ 6 M.

Bevorzugt liegt die Konzentrationen der chaotropen Verbindung des Lysereagenzes im Bereich von ≥ 3 M bis ≤ 7 M, besonders bevorzugt im Bereich von ≥ 4 M bis ≤ 6 M. Bevorzugt liegt die Konzentrationen der chaotropen Verbindung des Bindereagenzes im Bereich von ≥ 0,5 M bis ≤ 7 M, besonders bevorzugt im Bereich von ≥ 1 M bis ≤ 6 M. Bevorzugt liegt die Konzentrationen der chaotropen Verbindung des Waschreagenzes im Bereich von ≥ 0,5 M bis ≤ 3,5 M, besonders bevorzugt im Bereich von ≥ 0,9 M bis ≤ 3 M.

Gemäß einer weiter bevorzugten Ausführungsform umfasst das Lyse-, Binde- und/oder Waschreagenz wenigstens eine Pufferverbindung ausgewählt aus der Gruppe umfassend Tris(hydroxymethyl)aminomethan (TRIS), N-(2-Hydroxyethyl)piperazin-N'-(2-ethansulfonsäure) (HEPES), 3-(N-Morpholino)propan-sulfonsäure (MOPS) und/oder Phosphatpuffer.

Gemäß einer besonders bevorzugten Ausführungsform umfasst das Lyse-, Binde- und/oder Waschreagenz wenigstens eine Pufferverbindung ausgewählt aus der Gruppe umfassend Tris(hydroxymethyl)aminomethan (TRIS) und/oder N-(2-Hydroxyethyl)piperazin-N'-(2-ethansulfonsäure) (HEPES).

Das Lyse-, Binde- und/oder Waschreagenz ist vorzugsweise eine wässrige Lösung.

Gemäß einer weiter bevorzugten Ausführungsform weist das Lyse-, Binde- und/oder Waschreagenz einen pH-Wert im Bereich von ≥ 4 bis ≤ 12, insbesondere im Bereich von ≥ 6 bis ≤ 11, bevorzugt im Bereich von ≥ 7 bis ≤ 10 , besonders bevorzugt im Bereich von ≥ 8 bis ≤ 9 auf.

In bevorzugten Ausführungsformen kann das Lyse-, Binde- und/oder Waschreagenz , insbesondere das Lysereagenz, weiterhin Enzyme aufweisen, beispielsweise lytische Enzyme, insbesondere zum Beispiel Proteinase K, Protease (z.B. QIAGEN-Protease), Zymolase, Lyticase, Chromopeptidase, Lysostaphin, Lysozym, und, je nach Anwendung Nukleasen, zum Beispiel DNase und/oder RNase.

Erfindungsgemäße Lyse-, Binde- und/oder Waschreagenzien können Lysereagenzien, Bindereagenzien oder Waschreagenzien sein, oder Mischungen aus erfindungsgemäßen Lysereagenzien, Bindereagenzien und/oder Waschreagenzien.

Ein Immobilisieren von Nukleinsäuren an einer Matrix auf Basis einer oder mehrerer Siliciumoxidverbindung in Gegenwart einer chaotropen Verbindung erfolgt vorzugsweise in Anwesenheit eines verzweigten oder unverzweigten Alkanols. Daher umfasst zumindest das Bindereagenz vorzugsweise ein verzweigtes oder unverzweigtes Alkanol.

Bevorzugt verwendbar sind kurzkettige verzweigte oder unverzweigte Alkanole mit einem bis fünf Kohlenstoffatomen. Gemäß einer bevorzugten Ausführungsform der Erfindung ist das verzweigte oder unverzweigte Alkanol ein Alkohol mit einem bis fünf Kohlenstoffatomen, vorzugsweise ausgewählt aus der Gruppe umfassend Methanol, Ethanol, Isopropanol, n-Propanol, verzweigtes oder unverzeigtes Butanol oder Pentanol und/oder Mischungen davon.

Sofern nicht anders beschrieben, umfassen die Definitionen "verzweigtes oder unverzeigtes Alkanol" insbesondere Propanol, Butanol und Pentanol alle denkbaren isomeren Formen der jeweiligen Reste. So umfasst beispielsweise verzweigtes oder unverzeigtes Propanol n-Propanol und iso-Propanol, verzweigtes oder unverzeigtes Butanol umfasst iso-Butanol, sec-Butanol und tert-Butanol und verzweigtes oder unverzeigtes Pentanol umfasst beispielsweise n-Pentanol und iso-Pentanol. Bevorzugt werden Alkohole ausgewählt aus der Gruppe umfassend Methanol, Ethanol, Isopropanol und/oder deren Mischungen verwendet, besonders bevorzugt werden Alkohole ausgewählt aus der Gruppe umfassend Ethanol, Isopropanol und/oder deren Mischungen verwendet.

Gemäß einer bevorzugten Ausführungsform der Erfindung umfasst das Bindereagenz verzweigtes oder unverzweigtes Alkanol im Bereich von ≥ 20 Vol.-% bis ≤ 80 Vol.-%, vorzugsweise im Bereich von ≥ 40 Vol.-% bis ≤ 70 Vol.-%, bevorzugt im Bereich von ≥ 50 Vol.-% bis ≤ 60 Vol.-%, bezogen auf das Gesamtvolumen des Bindereagenzes.

Bei Angaben der Volumen- und/oder Gewichtsgehalte versteht es sich für den Fachmann von selbst, dass die Volumen- und/oder Gewichtsgehalte der einzelnen Komponenten so gewählt sind, dass das Gesamtvolumen oder Gesamtgewicht der Komponenten 100 Vol.-% oder 100 Gew.-% nicht übersteigt.

Die vorliegende Erfindung bezieht sich außerdem auf die Verwendung gemäß Anspruch 8, und demnach auf die Verwendung eines erfindungsgemäßen Lyse-, Binde- und/oder Waschreagenzes zur Isolierung und/oder Reinigung von Nukleinsäuren.

Die vorliegende Erfindung betrifft weiterhin ein Verfahren zur Isolierung und/oder Reinigung von Nukleinsäuren aus einer Nukleinsäuren enthaltenden biologischen Probe gemäß Anspruch 9, umfassend folgende Verfahrensschritte:
a) Lysieren der biologischen Probe,
b) Immobilisieren der freigesetzten Nukleinsäure(n) an einer Matrix auf Basis einer oder mehrerer Siliciumoxidverbindung(en) in Gegenwart einer chaotropen Verbindung und/oder eines verzweigten oder unverzweigten Alkanols,
c) optional Waschen der auf der Matrix immobilisierten Nukleinsäure(n),
d) optional Abtrennen der gebundenen Nukleinsäure,
wobei man das Lysieren und/oder Immobilisieren in Gegenwart einer Lyse- und/oder Bindezusammensetzung durchführt, umfassend:
- wenigstens eine chaotrope Verbindung, - wenigstens eine Pufferverbindung, und
- wenigstens ein polyoxyethylen-basiertes nicht-ionisches Tensid ausgewählt aus der Gruppe bestehend aus Polyoxyethylennonylphenylether, Polyoxyethylen(5)isooctylphenylether und Polyoxyethylen(12)isooctylphenylether im Bereich von ≥ 0,1 % Gewicht/Volumen bis ≤ 50 % Gewicht/Volumen, bezogen auf das Gesamtvolumen der Zusammensetzung.
Offenbart wird ein Verfahren zur Isolierung und/oder Reinigung von Nukleinsäuren aus einer Nukleinsäuren enthaltenden biologischen Probe, umfassend folgende Verfahrensschritte:
a) Lysieren der biologischen Probe,
b) Immobilisieren der freigesetzten Nukleinsäure(n) an einer Matrix auf Basis einer oder mehrerer Siliciumoxidverbindung(en) in Gegenwart einer chaotropen Verbindung und/oder eines verzweigten oder unverzweigten Alkanols,
c) optional Waschen der auf der Matrix immobilisierten Nukleinsäure(n),
d) optional Abtrennen der gebundenen Nukleinsäure,
wobei man das Lysieren und/oder Immobilisieren in Gegenwart einer Lyse- und/oder Bindezusammensetzung durchführt, umfassend:
- wenigstens eine chaotrope Verbindung, und
- wenigstens ein polyoxyethylen-basiertes nicht-ionisches Tensid ausgewählt aus der Gruppe umfassend Polyoxyethylen-Fettalkoholether, Polyoxyethylen-Alkylphenylether und/oder Polyoxyethylen-Polyoxypropylen Blockcopolymere im Bereich von ≥ 0,1 % (Gewicht/Volumen) bis ≤ 50 % (Gewicht/Volumen), bezogen auf das Gesamtvolumen der Zusammensetzung.

Unter dem Begriff "Zusammensetzung" werden im Sinne der vorliegenden Erfindung Lyse- und/oder Bindezusammensetzung verstanden.

In bevorzugten Ausführungsformen des Verfahrens verwendet man zum Lysieren der Probe ein erfindungsgemäßes Lysereagenz. Das Lysereagenz wird mit der zu lysierenden biologischen Probe in Kontakt gebracht. Ein oder mehrere Enzyme, können unabhängig voneinander, je nach Anwendung, zu verschiedenen Zeitpunkten zugesetzt werden. Die Probe kann in flüssiger Form vorliegen, beispielsweise im Fall flüssiger klinischer Proben. Klinische Proben, die feste Bestandteile enthalten, wie Stuhlproben oder Abstrichproben werden üblicherweise vor der weiteren Analyse in geeigneten wässrigen Lösungen suspspendiert. Zellkulturen werden meist vor der Lyse vom Kulturmedium abgetrennt, jedoch wird zumeist eine völlige Trocknung der Probe vermieden. Im Falle von völlig getrockneten Proben beispielsweise Lyophillisate wird die Probe vor der weiteren Verarbeitung in wässigen Lösungen rekonstituiert, beispielsweise Lyophillisate von Virus Standards. Daher enthalten die zu lysierenden Proben üblicherweise einen Anteil an Flüssigkeit. Diese in der Probe enthaltene Flüssigkeit wird mit dem Lysereagenz in Kontakt gebracht. Insofern liegt üblicherweise in einem Verfahren zur Isolierung und/oder Reinigung von Nukleinsäuren aus einer Probe eine Lysezusammensetzung vor, die Lysereagenz enthält sowie weitere Flüssigkeit der Probe oder bereits der Probe zugesetzter Lösungen.

Der Begriff "Lyse- und/oder Bindezusammensetzung" bezieht sich im Sinne der vorliegenden Erfindung auf ein Lyse- und/oder Bindereagenz, das in einem Verfahren zur Isolierung und/oder Reinigung von Nukleinsäuren aus einer Probe verwendet wird und neben Lyse-, Binde- und/oder Waschreagenz weitere Flüssigkeit enthalten kann. Die Lyse- und/oder Bindezusammensetzung kann vorzugsweise erfindungsgemäßes Lyse- und/oder Bindereagenz umfassen.

Gemäß einer weiter bevorzugten Ausführungsform des Verfahrens wird das Immobilisieren der freigesetzten Nukleinsäure an einer Matrix auf Basis einer oder mehrerer Siliciumoxidverbindung in Gegenwart einer erfindungsgemäßen Bindezusammensetzung durchgeführt.

Vorzugsweise wird erfindungsgemäßes Lyse- und/oder Bindereagenz mit der lysierten Probe in Kontakt gebracht. Die Lysezusammensetzung oder eine andere Lösung, in der das Lysieren durchgeführt wurde, kann vor dem in Kontakt Bringen mit dem Bindereagenz entfernt werden. Vorzugsweise wird die Lysezusammensetzung nicht entfernt. Bevorzugt wird ein Bindereagenz mit einer Probe umfassend Lysezusammensetzung in Kontakt gebracht.

Gemäß einer besonders bevorzugten Ausführungsform des Verfahrens wird das Lysieren in Gegenwart einer Lysezusammensetzung und das Immobilisieren in Gegenwart einer Bindezusammensetzung durchgeführt. Entsprechend wird das Immobilisieren vorzugsweise in Gegenwart einer Mischung einer Lyse- und Bindezusammensetzung durchgeführt.

Optional kann das Lysereagenz auch gleichzeitig als Bindereagenz dienen. Weiter optional kann das Bindereagenz auch als Lysereagenz dienen. Auch optional kann das Bindereagenz auch als Waschreagenz dienen.

Die Lyse- und/oder Bindezusammensetzung umfasst wenigstens eine chaotrope Verbindung, und wenigstens ein polyoxyethylen-basiertes nicht-ionisches Tensid ausgewählt aus der Gruppe bestehend aus Polyoxyethylennonylphenylether, Polyoxyethylen(5)isooctylphenylether und Polyoxyethylen(12)isooctylphenylether im Bereich von ≥ 0,1 % (Gewicht/Volumen) bis ≤ 50 % (Gewicht/Volumen), bezogen auf das Gesamtvolumen der Zusammensetzung. Für den Fall, dass Mischungen von Tensiden verwendet werden, handelt es sich bei den Konzentrationsangaben vorzugsweise um den Gesamtgehalt an Tensid beispielsweise im Bereich von insgesamt ≥ 0,1 % (Gewicht/Volumen) bis ≤ 50 % (Gewicht/Volumen), bezogen auf das Gesamtvolumen der Zusammensetzung.

Ein derartiges Verfahren bietet für die Isolierung und/oder Reinigung von Nukleinsäuren aus einer Nukleinsäuren enthaltenden biologischen Probe beispielsweise den Vorteil, dass bei Verwendung einer Lyse- und/oder Bindezusammensetzung umfassend wenigstens eine chaotrope Verbindung und wenigstens ein polyoxyethylen-basiertes nicht-ionisches Tensid, das ausgewählt ist aus der Gruppe bestehend aus Polyoxyethylennonylphenylether, Polyoxyethylen(5)isooctylphenylether und Polyoxyethylen(12)isooctylphenylether, im Bereich von ≥ 0,1 % (Gewicht/Volumen) bis ≤ 50 % (Gewicht/Volumen), bezogen auf das Gesamtvolumen der Zusammensetzung, auch nach mehrwöchiger oder mehrmonatiger Lagerung, bei Raumtemperatur oder erhöhten Temperaturen z.B. 50°C, ein Eluat enthaltend die Nukleinsäuren nicht oder nur geringfügig getrübt ist. In vorteilhafter Weise können somit keine oder zumindest deutlich weniger Verunreinigungen im Eluat enthalten sein. Hierdurch ist die weitere Verwendung des die Nukleinsäuren enthaltenden Eluats wesentlich vorteilhafter, da weitere zeitaufwendige und die Ausbeute an Nukleinsäuren verringernde Reinigungsschritte entfallen können.

Weiterhin bietet ein derartiges Verfahren für die Isolierung und/oder Reinigung von Nukleinsäuren aus einer Nukleinsäuren enthaltenden biologischen Probe beispielsweise den Vorteil, dass eine besonders gute Ausbeute an isolierten Nukleinsäuren insbesondere Virus-DNA beispielsweise der DNA von Hepatitis-B-Virus (HBV) ermöglicht wird.

Unter einer "biologischen Probe" kann ein Material auf partikulärer oder molekularer Basis verstanden werden, insbesondere Viren, Phagen und Zellen, wie Bakterienzellen, Hefe- oder Schimmelpilz-Zellen oder humane, tierische oder pflanzliche Zellen. Insbesondere eignet sich das Verfahren für die Isolierung von Nukleinsäuren wie DNA oder RNA aus Probenmaterialien humanen oder tierischen Ursprungs beispielsweise klinischer Proben, wie Blut, Plasma, Serum, Mund-, Rachen- und Nasenspülflüssigkeit, Broncheoalveoläre Lavagen, Urin, Zerebralflüssigkeit, Sputum, Speichel, Stuhl, Punktate, Abstriche, wie zum Beispiel Nasalabstriche, Wangenabstriche, Zervikalabstriche, Vaginalabstriche, Urethralabstriche, Pharyngealabstriche, Perinealabstriche, und Rektalabstriche, Stuhl, Punktate, Epithelabstriche, Biopsien und andere Gewebe- oder Knochenmarkproben, sowie Kulturen dieser Probenmaterialien in geeigneten Nährmedien.

Die Probe kann auch aus dem Bereich der Umweltanalytik, der Lebensmittelanalytik oder der molekularbiologischen Forschung stammen, beispielsweise aus Bakterienkulturen, Hefe- oder Pilzkulturen, Viruskulturen, Phagenlysaten oder Produkten von Amplifikationsverfahren, beispielsweise einer Polymerase-Kettenreaktion (PCR).

Das erfindungsgemäße Verfahren ist vorzugsweise geeignet zur Isolierung und/oder Reinigung von genomischer DNA, mitochondrialer DNA, Plasmid-DNA, von Virus-DNA und Virus-RNA und zur Isolierung und Aufreinigung von intrazellulärer RNA aus Vollblut beispielsweise für Reverse Transkription-Polymerase Kettenreaktion (Reverse Transkription -Polymerase Chain Reaction, RT-PCR), sowie zur Isolierung und/oder Aufreinigung von in zellfreien Probenmaterialien enthaltenen frei zirkuliernenden Nukleinsäuren. Das erfindungsgemäße Verfahren ist insbesondere für die Isolierung und/oder Aufreinigung von Virus-DNA geeignet.

In Schritt a) des Verfahrens erfolgt ein Lysieren der biologischen Probe. Grundsätzlich sind die nachstehend aufgeführten Methoden ausgewählt aus der Gruppe umfassend Lyse mit Hilfe von ionischen und nicht-ionogenen Tenside, beispielsweise Natriumdodecylsulfat (SDS), Lithiumdodecylsulfat (LiDS) oder Natriumlauroylsarkosinat (Sarkosyl) in geeigneten Reagenzien oder Puffern, die Verwendung von chaotropen Salzen, mechanisches Auseinanderreißen beispielsweise mittels Ultraschall, einer "french press", Mahlen mit Partikeln wie Glaskugeln, Keramikkugeln oder Metall-Partikeln oder in flüssigem Stickstoff, durch wiederholte Einfrier- und Auftauzyklen oder Kochen, enzymatische Lyse, Lyse durch Gefriertrocknen, Lyse durch osmotischen Schock, Mikrowellen- und/oder Temperaturbehandlung, und/oder Kombinationen davon zur Lyse biologischer Probe geeignet. Bevorzugt erfolgt die Lyse in Gegenwart chaotroper Salze.

Vorzugsweise erfolgt ein Lysieren der biologischen Probe in Gegenwart einer Lysezusammensetzung umfassend wenigstens eine chaotrope Verbindung und wenigstens ein polyoxyethylen-basiertes nicht-ionisches Tensid, das ausgewählt ist aus der Gruppe bestehend aus Polyoxyethylennonylphenylether, Polyoxyethylen(5)isooctylphenylether und Polyoxyethylen(12)isooctylphenylether, im Bereich von ≥ 0,1 % (Gewicht/Volumen) bis ≤ 50 % (Gewicht/Volumen), bezogen auf das Gesamtvolumen der Lysezusammensetzung.

Insbesondere ist eine Kombination aus Chaotropen und einem entsprechenden polyoxyethylen-basiertem nicht-ionischen Tensid für die Lyse von Virenzellen besonders effektiv.

Die Lyse- und/oder Bindezusammensetzung umfasst wenigstens eine chaotrope Verbindung, und wenigstens ein polyoxyethylen-basiertes nicht-ionisches Tensid, das ausgewählt ist aus der Gruppe bestehend aus Polyoxyethylennonylphenylether, Polyoxyethylen(5)isooctylphenylether und Polyoxyethylen(12)isooctylphenylether.

Es wird hierbei für die polyoxyethylen-basierten nicht-ionischen Tenside in vollem Umfang auf die vorstehende Beschreibung Bezug genommen.

Die Lyse- und/oder Bindezusammensetzung umfasst wenigstens ein polyoxyethylen-basiertes nicht-ionisches Tensid. Offenbart wird, dass Polyoxyethylenalkylphenylether eine Alkylgruppe mit fünf bis 15 Kohlenstoffatomen, oder mit 6 bis 10 Kohlenstoffatomen, aufweisen können. Weiter offenbart sind verzweigte oder unverzweigte C₇- bis C₁₀-Alkylgruppen, insbesondere verzweigte oder unverzweigte C₈- und C₉-Alkylgruppen, besonders Isooctyl- und Nonyl-Gruppen. In der beanspruchten Ausführungsform des Verfahrens ist der Polyoxyethylenalkylphenylether ausgewählt aus der Gruppe bestehend aus Polyoxyethylennonylphenylether, Polyoxyethylen(5)isooctylphenylether und Polyoxyethylen(12)isooctylphenylether.Polyoxyethylennonylphenylether und Polyoxyethylenisooctylphenylether sind beispielsweise erhältlich unter der Handelsbezeichnung Igepal®, beispielsweise von der Firma BASF.

Gemäß einer bevorzugten Ausführungsform des Verfahrens umfasst die Lyse- und/oder Bindezusammensetzung nicht-ionisches Tensid im Bereich von ≥ 0,2 % (Gewicht/Volumen) bis ≤ 30 % (Gewicht/Volumen), vorzugsweise im Bereich von ≥ 3 % (Gewicht/Volumen) bis ≤ 10 % (Gewicht/Volumen), bevorzugt im Bereich von ≥ 3,2 % (Gewicht/Volumen) bis ≤ 8 % (Gewicht/Volumen), bezogen auf das Gesamtvolumen der Zusammensetzung.

Dies hat sich insbesondere für Lysezusammensetzungen und für Mischungen von Lyse- und Bindezusammensetzungen als günstig herausgestellt.

Gemäß einer bevorzugten Ausführungsform des Verfahrens ist die chaotrope Verbindung der Lyse- und/oder Bindezusammensetzung ein Natrium- oder Guanidiniumsalz, vorzugsweise ausgewählt aus der Gruppe umfassend Natriumiodid, Natriumperchlorat, Guanidiniumhydrochlorid, Guanidiniumthiocyanat, Guanidiniumisothiocyanat und/oder eine Mischung zweier oder mehrerer Salze davon. Bevorzugt ist die chaotrope Verbindung ein Guanidiniumsalz, vorzugsweise ausgewählt aus der Gruppe umfassend Guanidiniumhydrochlorid, Guanidiniumthiocyanat und/oder Guanidiniumisothiocyanat.

Insbesondere hat sich eine Kombination der vorgenannten chaotropen Verbindungen und der erfindungsgemäß enthaltenen polyoxyethylen-basierten nicht-ionischen Tensidien für die Lyse von Viruszellen und die Isolierung von Nukleinsäuren aus Viruszellen als günstig erwiesen.

Konzentrationen der chaotropen Verbindung der Lyse- und/oder Bindezusammensetzung im Bereich von ≥ 0,1 M bis ≤ 10 M haben sich als günstig erwiesen. Vorzugsweise liegt die Konzentrationen der chaotropen Verbindung im Bereich von ≥ 1 M bis ≤ 8 M, bevorzugt im Bereich von ≥ 3 M bis ≤ 7 M, besonders bevorzugt im Bereich von ≥ 4 M bis ≤ 6 M.

Gemäß einer bevorzugten Ausführungsform des Verfahrens umfasst die Lyse- und/oder Bindezusammensetzung wenigstens eine Pufferverbindung ausgewählt aus der Gruppe umfassend Tris(hydroxymethyl)aminomethan (TRIS), N-(Tri(hydroxymethyl)methyl)glycin (Tricin), N,N-Bis(2-hydroxyethyl)-glycin (BICIN), N-(2-Hydroxyethyl)piperazin-N'-(2-ethansulfonsäure) (HEPES), Piperazin-1,4-bis(2-ethansulfonsäure) (PIPES), N-Cyclohexyl-2-aminoethansulfonsäure (CHES), 2-(N-Morpholino)-ethansulfonsäure (MES), 3-(N-Morpholino)propansulfonsäure (MOPS) und/oder Phosphatpuffer.

Gemäß einer weiter bevorzugten Ausführungsform des Verfahrens umfasst die Lyse- und/oder Bindezusammensetzung wenigstens eine Pufferverbindung ausgewählt aus der Gruppe umfassend Tris(hydroxymethyl)aminomethan (TRIS), N-(2-Hydroxyethyl)piperazin-N'-(2-ethansulfonsäure) (HEPES), und/oder Phosphatpuffer. Gemäß einer noch weiter bevorzugten Ausführungsform des Verfahrens umfasst die Lyse- und/oder Bindezusammensetzung wenigstens eine Pufferverbindung ausgewählt aus der Gruppe umfassend Tris(hydroxymethyl)aminomethan (TRIS) und/oder N-(2-Hydroxyethyl)piperazin-N'-(2-ethansulfonsäure) (HEPES).

Das Lysieren der biologischen Probe kann bei Raumtemperatur beispielsweise bei 15°C bis 25°C oder bei erhöhter Temperatur, beispielsweise bei Temperaturen im Bereich von ≥ 37°C bis ≤ 75°C erfolgen.

In bevorzugten Ausführungsformen kann die Lysezusammensetzung weiterhin Enzyme aufweisen, beispielsweise Proteinase K, Protease (z.B. QIAGEN-Protease), Zymolase, Lyticase, Achromopeptidase, Lysostaphin, Lysozym, und, je nach Anwendung Nukleasen, zum Beispiel DNase und/oder RNase.

Das Immobilisieren der freigesetzten Nukleinsäure(n) an einer Matrix auf Basis einer oder mehrerer Siliciumoxidverbindung erfolgt in Gegenwart einer chaotropen Verbindung und/oder eines verzweigten oder unverzweigten Alkanols.

Vorzugsweise umfasst die Bindezusammensetzung ein verzweigtes oder unverzweigtes Alkanol. Gemäß einer bevorzugten Ausführungsform ist das verzweigte oder unverzweigte Alkanol ein Alkohol mit einem bis fünf Kohlenstoffatomen, vorzugsweise ausgewählt aus der Gruppe umfassend Methanol, Ethanol, Isopropanol, n-Propanol, n-Butanol, iso-Butanol, sec-Butanol, tert- Butanol, n-Pentanol, iso-Pentanol und/oder Mischungen davon.

Gemäß einer bevorzugten Ausführungsform umfasst die Bindezusammensetzung verzweigtes oder unverzweigtes Alkanol im Bereich von ≥ 1 Vol.-% bis ≤ 80 Vol.-%, vorzugsweise im Bereich von ≥ 5 Vol.-% bis ≤ 70 Vol.-%, bevorzugt im Bereich von ≥ 10 Vol.-% bis ≤ 60 Vol.-%, weiter vorzugsweise im Bereich von ≥ 15 Vol.-% bis ≤ 50 Vol.-%, bezogen auf das Gesamtvolumen der Bindezusammensetzung.

Gemäß einer bevorzugten Ausführungsform der Erfindung umfasst eine Mischung der Bindezusammensetzung das Lysereagenz und optional ein oder mehrere weitere Zusätze bevorzugt verzweigtes oder unverzweigtes Alkanol im Bereich von ≥ 1 Vol.-% bis ≤ 80 Vol.-%, vorzugsweise im Bereich von ≥ 5Vol.-% bis ≤ 70Vol.-%, bevorzugt im Bereich von ≥ 15 Vol.-% bis ≤ 50 Vol.-%, bezogen auf das Gesamtvolumen der Mischung.

Zur Isolierung der Nukleinsäuren wird die Probe mit einer Matrix auf Basis einer oder mehrerer Siliciumoxidverbindungen, wie Siliziumdioxid (Silica), Silicat, Glas und/oder Silicagel in Kontakt gebracht und für eine für die Bindung ausreichende Zeit inkubiert. Die Matrix kann in den üblichen aus dem Stand der Technik bekannten Ausgestaltungen vorliegen wie zum Beispiel in Form von Partikeln, als Membran oder Filter. Zur leichteren Abtrennung weisen die Partikel vorzugsweise magnetische Eigenschaften auf. Für Nukleinsäuren können Inkubationszeiten zwischen 10 Sekunden und 30 Minuten zweckmäßig sein. Es haben sich Inkubationszeiten in einem Bereich von 1 Minute bis 20 Minuten, insbesondere von ca. 10 Minuten als vorteilhaft erwiesen.

Zum Isolieren von Nukleinsäuren werden bevorzugt magnetische Partikel verwendet, die eine Kieselgelhülle aufweisen. Zum Isolieren von Nukleinsäuren werden bevorzugt magnetische Partikel verwendet, die eine Kieselgelhülle aufweisen und die eine mittlere Partikelgröße im Bereich von ≥ 1 µm bis ≤ 25 µm, vorzugsweise im Bereich von ≥ 5 µm bis ≤ 15 µm und besonders bevorzugt im Bereich von ≥ 6 µm bis ≤ 10 µm, vorzugsweise mit einer engen Größenverteilung, aufweisen. Zum Isolieren von Nukleinsäuren werden weiter bevorzugt magnetische Partikel verwendet, die eine Kieselgelhülle aufweisen und die eine mittlere Partikelgröße im Bereich von ≥ 1 µm bis ≤ 5 µm, vorzugsweise mit einer engen Größenverteilung, aufweisen.

In einer weiter bevorzugten Ausführungsform sind die magnetischen oder magnetisch anziehbaren Partikel Partikel, die einen magnetischen auf Eisenoxid basierenden Kern, vorzugsweise ausgewählt aus der Gruppe umfassend Magnetit (Fe₃O₄), Maghemit (γ-Fe₂O₃) und/oder Ferriten, aufweisen.

Magnetische Silicapartikel, die in vorteilhafter Weise verwendet werden können, sind beispielsweise in der internationalen Patentanmeldung WO 01/71732 beschrieben.

In einer bevorzugten Ausführungsform ist eine Matrix auf Basis einer oder mehrerer Siliciumoxidverbindung in Form magnetischer oder magnetisch anziehbarer Partikel mit einer Silica-Oberfläche verwendbar.

Vorzugsweise erfolgt die Bindung bei Temperaturen im Bereich von ≥ 15°C bis ≤ 75 °C, bevorzugt im Bereich von ≥ 20°C bis ≤ 70 °C, besonders bevorzugt im Bereich von ≥ 46°C bis ≤ 65 °C am meisten bevorzugt von ≥ 50°C bis ≤ 60°C Die Bindung kann auch bei Raumtemperatur beispielsweise bei ≥ 15°C bis ≤ 28°C erfolgen.

Nach der Inkubation werden die an die Matrix auf Basis einer oder mehrerer Siliciumoxidverbindung gebundenen Nukleinsäuren von der Lyse- und/oder Bindezusammensetzung abgetrennt. Bei Verwendung von magnetischen Silicapartikeln kann dies mit Hilfe eines Magnetfeldes erreicht werden. Beispielsweise können die Magnetpartikel an die Wand des Gefäßes, in dem die Inkubation stattgefunden hatte, gezogen werden, in geeigneten Pipettenspitzen durch Anlegen eines Magnetfeldes gesammelt werden, oder an durch Kunststoffhüllen geschützten MagnetStäben immobilisiert werden. Geeignete Verfahrensschritte zum Entfernen der Lyse- und/oder Bindezusammensetzung sind beispielsweise Abpipettieren oder Absaugen der Flüssigkeit, oder das Herausheben der Magnetpartikel in Pipettenspitzen oder an Magnetstäben bzw. das Absenken des Lyse- und/oder Bindeansatzes wobei die separierten Magnetpartikel auf gleicher Höhe verbleiben.

Optional können die auf der Matrix immobilisierten Nukleinsäure(n) vor dem Abtrennen gewaschen werden. Der Waschschritt findet bevorzugt durch Inkubation einer Waschlösung mit den beladenen Partikeln statt, wobei bevorzugt eine Resuspension der Partikel erfolgt, beispielsweise durch Schütteln oder Anlegen eines Magnetfeldes. Die verunreinigte Waschlösung wird vorzugsweise ebenso wie die nach der Bindung verbleibende Lyse- und/oder Bindezusammensetzung, insbesondere ein Gemisch von Lyse- und/oder Bindezusammensetzung entfernt.

Als Waschreagenz kann ein herkömmlicher Waschpuffer oder jedes andere, geeignete Medium verwendet werden. Im Allgemeinen werden Waschreagenzien mit niedriger bis moderater Ionenstärke bevorzugt, beispielsweise eine Lösung von 10 mM Tris(hydroxymethyl)aminomethan (TRIS). Weiterhin sind Waschpuffer, die höhere Konzentrationen an Salzen aufweisen beispielsweise eine Lösung von 4-6 M Guanidinium-Hydrochlorid verwendbar. Die zuvor beschriebenen erfindungsgemäßen Waschreagenzien stellen ebenfalls geeignete Waschreagenzien dar.

Weiterhin sind alkoholhaltige Waschreagenzien verwendbar, beispielsweise wässrige Lösungen von Alkoholen mit eins bis fünf Kohlenstoffatomen, bevorzugt wässrige Lösungen von Ethanol, insbesondere wässrige Lösungen von 50-100 prozentigem Ethanol.

Vorzugsweise werden die auf der Matrix immobilisierten Nukleinsäure(n) mehrmals beispielsweise 2fach bis 4fach, gewaschen, bevorzugt mit verschiedenen Waschreagenzien. In bevorzugten Ausführungsformen erfolgt das Waschen zunächst mit Waschreagenzien mit niedriger bis moderater Ionenstärke, und anschließend mit einer wässrigen 70-100 prozentigen Lösung von Ethanol.

Insbesondere eine Verwendung magnetischer Partikel ermöglicht eine einfache Durchführung von Separations- und/oder Waschschritten durch die magnetische Aggregation der Partikel.

Im Anschluss an den letzten Waschschritt oder einem Wasserspülschritt ("water rinse") kann ein Trocknungsschritt der vorzugsweise magnetischen Partikel beispielsweise im Vakuum oder durch Ausdampfen oder Ausdampfen lassen der Flüssigkeit vorgenommen werden.

Gemäß Schritt d) des Verfahrens können die gebundenen Nukleinsäuren von der Matrix abgetrennt werden. Das Abtrennen der Nukleinsäuren wird auch als Eluieren bezeichnet.

Es kann auch bevorzugt sein, die an die Matrix insbesondere an magnetische Partikel gebundenen Nukleinsäuren ohne Abtrennung zu verwenden, beispielsweise für PCR oder anderen Amplifikationsmethoden, DNA-Detektionsverfahren oder DNA-Identifikationsverfahren.

Die gebundene Nukleinsäure kann mittels eines Elutionsreagenz mit niedrigem Salzgehalt von den Partikeln abgetrennt werden. Als Elutionsreagenz mit niedrigem Salzgehalt sind insbesondere Reagenzien mit einem Salzgehalt von weniger als 0,1 mol/l verwendbar. Besonders bevorzugt enthält das Elutionsreagenz die Pufferverbindung Tris(hydroxymethyl)aminomethan (Tris). Weiterhin besonders geeignet zur Elution ist demineralisiertes Wasser, optional mit einem oder mehreren Zusätzen, beispielsweise Komplexbildner wie Ethylendiamin-tetraacetat (EDTA), Azid und/oder Pufferverbindungen beispielsweise Tris(hydroxymethyl)aminomethan (Tris).

Insbesondere durch Verwendung der Lyse- und/oder Bindezusammensetzung ergibt sich ein besonders vorteilhaftes Verfahren zur Isolierung von Nukleinsäuren aus biologischen Proben, insbesondere zur Isolierung von Virus-DNA.

Vorteile ergeben sich insbesondere aus den erzielbaren guten Ausbeuten auch nach Lagerung der Lyse- und/oder Bindereagenzien.

Die vorliegende Erfindung betrifft weiterhin ein Kit gemäß Anspruch 13, und demnach ein Kit zur Isolierung und/oder Reinigung von Nukleinsäuren aus einer Nukleinsäuren enthaltenden biologischen Probe, umfassend ein erfindungsgemäßes Lyse-, Binde- und/oder Waschreagenz.

In bevorzugten Ausführungsformen kann das Kit weiterhin eine Matrix auf Basis einer oder mehrerer Siliciumoxidverbindung enthalten, insbesondere eine Matrix auf Basis einer oder mehrerer Siliciumoxidverbindung in Form magnetischer oder magnetisch anziehbarer Partikel mit einer Silica-Oberfläche. Bevorzugt enthaltene magnetische Silicapartikel, sind beispielsweise in der internationalen Patentanmeldung WO 01/71732 beschrieben.

In einer weiter bevorzugten Ausführungsform kann das Kit weiterhin geeignete Wasch- und/oder Elutionsreagenzien enthalten, insbesondere erfindungsgemäßes Waschreagenz.

In einer weiteren bevorzugten Ausführungsform kann das Kit statt magnetischen Silikapartikeln andere silanisierte Träger-Materialien bevorzugt Zentrifugensäulchen mit Silika-Membranen enthalten.

Offenbart wird die Verwendung von polyoxyethylen-basierten nicht-ionischen Tensiden ausgewählt aus der Gruppe umfassend Polyoxyethylen-Fettalkoholether, Polyoxyethylen-Alkylphenylether und/oder Polyoxyethylen-Polyoxypropylen Blockcopolymere insbesondere Polyoxyethylen-Fettalkoholethern ausgewählt aus der Gruppe umfassend Polyoxyethylenlaurylether, Polyoxyethylencetylether, Polyoxyethylenstearylether und/oder Polyoxyethylenoleylether zur Solubilisierung von Lipiden einer biologischen Probe.

Unter dem Begriff "Lipid" sind im Sinne der vorliegenden Erfindung wasserunlösliche oder zumindest größtenteils wasserunlösliche Naturstoffe zu verstehen. Der Begriff "Lipid" umfasst im Sinne der vorliegenden Erfindung Fettsäuren, die Gruppe der Triglyceride umfassend Fette und Öle, Wachse, Phospholipide, Sphingolipide, Liposaccharide und die Gruppe der Isoprenoide umfassend Steroide und Carotinoide. Insbesondere sind unter dem Begriff "Lipid" Lipidbestandteile oder Strukturkomponenten der Zellmembranen von Organismen wie Phospholipide und Sphingolipide zu verstehen.

Bevorzugt ist eine Verwendung von polyoxyethylen-basierten nicht-ionischen Tensiden ausgewählt aus der Gruppe umfassend Polyoxyethylen-Fettalkoholether, Polyoxyethylen-Alkylphenylether und/oder Polyoxyethylen-Polyoxypropylen Blockcopolymere zur Solubilisierung von Lipiden einer biologischen Probe bei Verfahren zur Isolierung und/oder Reinigung von Nukleinsäuren aus einer Nukleinsäuren enthaltenden biologischen Probe.

Es wird hierbei für die polyoxyethylen-basierten nicht-ionischen Tensidien in vollem Umfang auf die vorstehende Beschreibung Bezug genommen.

Insbesondere bevorzugt ist eine Verwendung von Polyoxyethylen-Fettalkoholethern ausgewählt aus der Gruppe umfassend Polyoxyethylenlaurylether, Polyoxyethylencetylether, Polyoxyethylenstearylether und/oder Polyoxyethylenoleylether zur Solubilisierung von Lipiden einer biologischen Probe bei Verfahren zur Isolierung und/oder Reinigung von Nukleinsäuren aus einer Nukleinsäuren enthaltenden biologischen Probe.

Besonders bevorzugt ist eine Verwendung der polyoxyethylen-basierten nicht-ionischen Tensidien insbesondere Polyoxyethylen-Fettalkoholethern zur Solubilisierung von Lipiden einer biologischen Probe bei Verfahren zur Isolierung und/oder Reinigung von Nukleinsäuren unter Verwendung einer Matrix auf Basis einer oder mehrerer Siliciumoxidverbindung vorzugsweise in Form magnetischer oder magnetisch anziehbarer Partikel mit einer Silica-Oberfläche.

In vorteilhafter Weise konnte festgestellt werden, dass bei einer Verwendung der polyoxyethylen-basierten nicht-ionischen Tensiden insbesondere Polyoxyethylen-Fettalkoholethern bei Verfahren zur Isolierung und/oder Reinigung von Nukleinsäuren unter Verwendung einer Matrix auf Basis einer oder mehrerer Siliciumoxidverbindung vorzugsweise in Form magnetischer oder magnetisch anziehbarer Partikel mit einer Silica-Oberfläche keine oder zumindest deutlich weniger Verunreinigungen im Eluat enthalten waren.

Darüber hinaus ist die Erfindung gerichtet auf die Verwendung gemäß Anspruch 14, und demnach die Verwendung von polyoxyethylen-basierten nicht-ionischen Tensiden ausgewählt aus der Gruppe bestehend aus Polyoxyethylennonylphenylether, Polyoxyethylen(5)isooctylphenylether und Polyoxyethylen(12)isooctylphenylether, zur Herstellung erfindungsgemäßer lagerstabiler Binde-, Lyse- und/oder Waschreagenzien.

Offenbart wird die Verwendung von polyoxyethylen-basierten nicht-ionischen Tensiden ausgewählt aus der Gruppe umfassend Polyoxyethylen-Fettalkoholether, Polyoxyethylen-Alkylphenylether und/oder Polyoxyethylen-Polyoxypropylen Blockcopolymere, vorzugsweise Polyoxyethylen-Fettalkoholether ausgewählt aus der Gruppe umfassend Polyoxyethylenlaurylether, Polyoxyethylencetylether, Polyoxyethylenstearylether und/oder Polyoxyethylenoleylether, zur Herstellung lagerstabiler Binde-, Lyse- und/oder Waschreagenzien.

Es wird hierbei für die polyoxyethylen-basierten nicht-ionischen Tenside in vollem Umfang auf die vorstehende Beschreibung Bezug genommen.

Unter "lagerstabil" ist dabei im Sinne der Erfindung vorzugsweise zu verstehen, dass sich die für die jeweilige Anwendung relevanten Eigenschaften des Lyse-, Bindungs- oder Waschreagenzes bei Lagerung in einem Zeitraum von drei Monaten, vorzugsweise von 6 Monaten, weiter bevorzugt von mindestens 8 Monaten nicht derart ändern, dass die Anwendung dadurch wesentlich beeinträchtigt wird. In einer bevorzugten Ausführungsform zeigt sich die Lagerstabilität bei Raumtemperatur, weiter bevorzugt auch bei einer erhöhten Lagertemperatur von beispielsweise 50°C.

Als eine der relevanten Eigenschaften der Reagenzien für die Anwendung hat sich der pH-Wert herausgestellt. Vorzugsweise ändert sich dieser daher bei der Lagerung der Reagenzien nicht wesentlich, bevorzugt wird der pH-Wert bei der Lagerung der Reagenzien um weniger als 1 abgesenkt.
- Fig. 1a, 1b: zeigt die Änderung des pH-Wertes des Lysereagenzes B, dargestellt als ungefüllte Balken, und des Tween 20®-haltigen Lysereagenzes A, dargestellt als gefüllte Balken, während 33 Wochen Lagerung bei 25°C (Fig. 1a) und 50°C (Fig. 1b).
- Fig. 2: zeigt die Mittelwerte der CT-Werte nach HBV-spezifischer real-time-PCR von HBV-DNA nach Durchführung von Präparationen viraler DNA mit dem Lysereagenz B und dem Tween® 20-haltigen Lysereagenz A.
- Fig. 3: zeigt die Mittelwerte der CT-Werte nach HBV-spezifischer real-time-PCR von HBV-DNA nach Durchführung von Präparationen viraler DNA mit dem Lysereagenz B und dem Tween® 20-haltigen Lysereagenz A nach 10 Wochen Lagerung bei 50°C. Hierbei diente ein Lysereagenz A, das etwa 4 Wochen bei Raumtemperatur gelagert wurde, als Referenz. Es wurden jeweils 6 µl und 24 µl des Eluats für die real-time-PCR verwendet, wobei die Ergebnisse für 6 µl des Eluats dargestellt sind als ungefüllte Balken und die für 24 µl Eluat dargestellt sind als gefüllte Balken.

Beispiele, die sich nicht auf den beanspruchten Gegenstand beziehen, dienen lediglich der Illustration.

### Beispiel 1: Stabilitätsuntersuchung

Ein Lysereagenz A enthaltend 20 % (w/v) Tween® 20 (Fa. Fluka), Guanidiniumisothiocyanat und Tris(hydroxymethyl)aminomethan und Lysereagenz B , bei dem das Tween® 20 durch 20 % (w/v) Brij® 58 (Fa. Sigma) ausgetauscht wurde, wurden in bidestilliertem Wasser frisch angesetzt und jeweils in geschlossenen Gefäßen für 33 Wochen bei 25°C und 50°C gelagert.

Hierbei wurde zum Zeitpunkt der Einlagerung sowie in wöchentlichen Intervallen jeweils der pH-Wert der Lösungen mit Hilfe eines pH-Meters (Firma Metrohm) bei Temperaturen im Bereich von 20°C bis 28°C bestimmt.

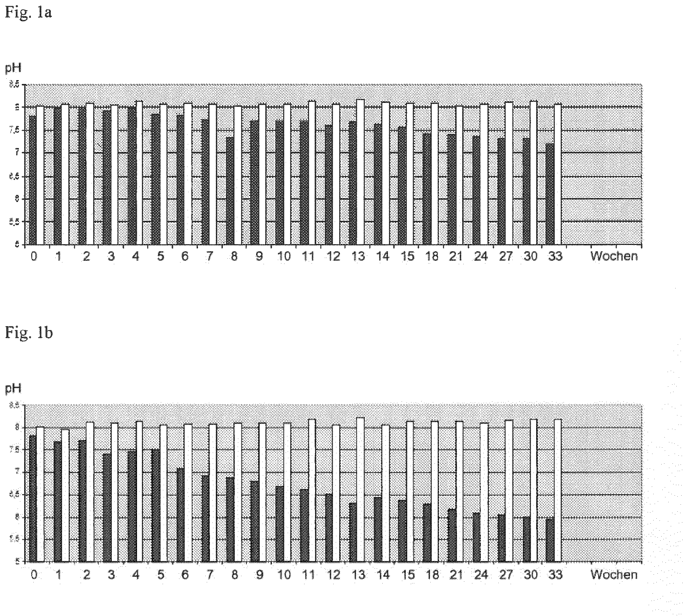

Anhand des in Fig. 1a dargestellten Balkendiagramms ist ersichtlich, dass der pH-Wert des Lysereagenzes A während 33 Wochen Lagerung bei 25°C von ca. pH 7,8 zu pH 7,2 leicht abnahm, während der pH-Wert während 33 Wochen Lagerung bei 50°C von ca. pH 7,8 zu ca. pH 5,9 abnahm, wie in Fig. 1b dargestellt ist. Demgegenüber blieb der pH-Wert des Lysereagenzes B während der 33 Wochen Lagerung bei 25°C und 50°C stabil bei ca. pH 8.

### Beispiel 2: Extraktion viraler DNA

Negatives, d.h. HBV-Virus-freies, Humanplasma wurde mit 10⁴ sgU/ml Hepatitis-B-Virus (HBV) versetzt. Aus jeweils 1000 µl der Plasmaprobe wurde die virale DNA unter Verwendung der kommerziell erhältlichen Automationsplattform QIAsymphony® (Fa. Qiagen) mittels des automatisierten Protokolls zur Aufreinigung von viraler Nukleinsäure aus Plasmaproben extrahiert.

Gemäß dem verwendeten Protokoll wurde die Probe mit den im Protokoll definierten Volumina - an Lysereagenz B enthaltend Guanidiniumisothiocyanat, Tris(hydroxymethyl)aminomethan und 20 % (w/v) Brij® 58 (Fa. Sigma) und Proteinase K und Lösung AVE enthaltend Carrier-RNA in Kontakt gebracht. Es erfolgte eine Inkubation bei 65°C zur Lyse der Probe. Anschließend wurde dem Probenansatz das im Protokoll definierte Volumen an Bindereagenz C enthaltend Guanidiniumisothiocyanat, Tris(hydroxymethyl)aminomethan und 9 % (w/v) Brij® 58 (Fa. Sigma) sowie Isopropanol zugegeben. Nach einer weiteren 3 minütigen Inkubation wurde MagAttract Suspension , welche die magnetischen Siliciumoxid-Partikel enthielt, zugegeben und wie im Protokoll vorgesehen vermischt. Während dieser Zeit binden die Nukleinsäuren an die Siliciumoxid-Partikel. Anschließend wurden die magnetischen Siliciumoxid-Partikel separiert und die flüssige Phase entfernt. Zu den Siliciumoxid-Partikeln wurden anschließend das im Protokoll definierte Volumen an Waschlösung enthaltend Guanidiniumthiocyanat und Ethanol zugeben und die Partikel in der Waschlösung suspendiert. Es erfolgte erneutes Entfernen des Überstandes sowie Zugabe von im Protokoll definiertem Volumen an Waschlösung enthaltend Tris, NaCl und Ethanol und ein zweiter Waschschritt. Nach dem Separieren und Abtrennen der flüssigen Phase wurden die Partikel mit im Protokoll definierte Volumen an wässrigem 80%igem Ethanol gewaschen. Nach dem Separieren der Partikel wurde der Überstand entfernt und es erfolgte Lufttrocknen der Partikel für 8 Minuten. Um die DNA zu eluieren wurden im Protokoll definierte Volumen an Elutionslösung E zugegeben und die Partikel für 3 Minuten darin suspendiert. Anschließend wurden die Partikel entfernt und das Eluat erhalten.

Gemäß diesem Protokoll wurde aus einer weiteren 1000 µl Plasmaprobe virale DNA extrahiert, wobei abweichend Lysereagenz A enthaltend 20 % (w/v) Tween® 20 (Fa. Fluka) und Bindereagenz D enthaltend 9 % (w/v) Tween® 20 (Fa. Fluka) verwendet wurden.

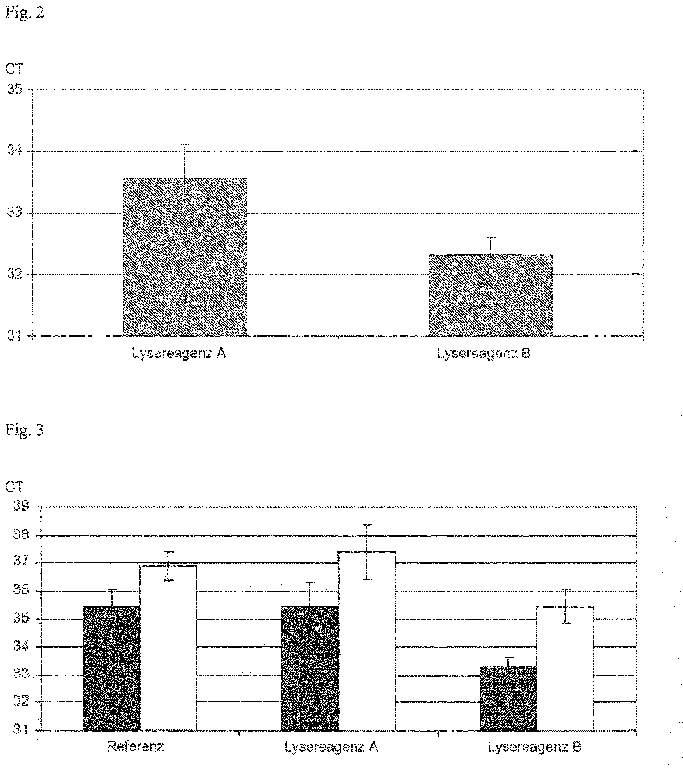

Die erhaltenen Eluate wurden jeweils einer HBV-spezifischen real-time (RT-)-PCR unterzogen, wobei jeweils 24 µl Eluat verwendet wurden. Aus den in Fig. 2 dargestellten Mittelwerten der CT-Werte (Threshold Cycle, "Schwellenwert-Zyklus"), die den Zyklus beschreiben, an dem die Fluoreszenz beginnt logarithmisch zuzunehmen, ist erkennbar, dass die Extraktion unter Verwendung des Lysereagenzes B und Bindereagenzes C eine höhere Ausbeute erzielte.

### Beispiel 3: Extraktion viraler DNA nach Lagerung des Lysereagenzes

Die Lysereagenzien B enthaltend Guanidiniumisothiocyanat, Tris(hydroxymethyl)aminomethan und 20 % (w/v) Brij® 58 (Fa. Sigma) und A, in dem das Brij® 58 durch 20 % (w/v) Tween® 20 (Fa. Fluka) ausgetauscht war, wurden jeweils in geschlossenen Gefäßen für 10 Wochen bei 50°C gelagert.

Anschließend wurde unter Verwendung der kommerziell erhältlichen Automationsplattform QIAsymphony® (Fa. Qiagen) mittels des automatisierten Protokolls zur Aufreinigung von viraler Nukleinsäure aus Plasmaproben die virale Nukleinsäure extrahiert.

Gemäß dem in Beispiel 2 beschriebenen Protokoll wurde aus jeweils 1000 µl der Plasmaprobe die virale DNA unter Verwendung der kommerziell erhältlichen Automationsplattform QIAsymphony® (Fa. Qiagen) extrahiert, wobei für unterschiedliche Ansätze jeweils für 10 Wochen bei 50°C gelagerter Lysereagenz B enthaltend Guanidiniumisothiocyanat, Tris(hydroxymethyl)aminomethan und 20 % (w/v) Brij® 58 (Fa. Sigma) und Lysereagenz A, in dem das Brij® 58 durch 20 % (w/v) Tween® 20 (Fa. Fluka) ausgetauscht war. Als Referenz diente ein Lysereagenz A, der etwa 4 Wochen bei Raumtemperatur gelagert wurde.

Es wurde festgestellt, dass die Eluate, die unter Verwendung des bei 50°C gelagerten Lysereagenzes A erhalten wurden, stark getrübt waren, während die Eluate, die unter Verwendung des Lysereagenzes B erhalten wurden, klar waren.

Von den erhaltenen Eluaten wurden jeweils 6 µl und 24 µl einer HBV-spezifischen real-time (RT-)-PCR unterzogen. Aus den in Fig. 3 dargestellten Mittelwerten der CT-Werte ist erkennbar, dass die Extraktion unter Verwendung der Lysereagenz B und Bindereagenz C eine höhere Ausbeute erzielte.

## Patentansprüche

1. Lyse-, Binde- und/oder Waschreagenz umfassend:
- wenigstens eine chaotrope Verbindung,
- wenigstens eine Pufferverbindung vorzugsweise ausgewählt aus der Gruppe umfassend Tris(hydroxymethyl)aminomethan, N-(Tri(hydroxymethyl)methyl)glycin, N,N-Bis(2-hydroxyethyl)-glycin, 3-(N-Morpholino)propansulfonsäure, N-(2-Hydroxyethyl)piperazin-N'-(2-ethansulfonsäure), Piperazin-1,4-bis(2-ethansulfonsäure), N-Cyclohexyl-2-aminoethansulfonsäure, 2-(N-Morpholino)-ethansulfonsäure und/oder Phosphatpuffer, und
- wenigstens ein polyoxyethylen-basiertes nicht-ionisches Tensid ausgewählt aus der Gruppe bestehend aus Polyoxyethylennonylphenylether, Polyoxyethylen(5)isooctylphenylether und Polyoxyethylen(12)isooctylphenylether im Bereich von ≥ 8 % Gewicht/Volumen bis ≤ 50 % Gewicht/Volumen, bezogen auf das Gesamtvolumen des Reagenzes.

2. Lyse-, Binde- und/oder Waschreagenz nach Anspruch 1, **dadurch gekennzeichnet, dass** das polyoxyethylen-basierte nicht-ionische Tensideinen Polyoxyethylen-Bestandteil umfasst, der 2 bis 150 (CH₂CH₂O)-Einheiten enthält.

3. Lyse-, Binde- und/oder Waschreagenz nach Anspruch 1 oder 2, **dadurch gekennzeichnet, dass** der Polyoxyethylennonylphenylether ausgewählt ist aus der Gruppe bestehend aus Polyoxyethylen(2)nonylphenylether, Polyoxyethylen(5)nonylphenylether, Polyoxyethylen(9)nonylphenylether, Polyoxyethylen(12)nonylphenylether und Polyoxyethylen(100)nonylphenylether.

4. Lyse-, Binde- und/oder Waschreagenz nach einem der vorherigen Ansprüche, **dadurch gekennzeichnet, dass** der Polyoxyethylennonylphenylether ausgewählt ist aus der Gruppe bestehend aus Polyoxyethylen(5)nonylphenylether, Polyoxyethylen(9)nonyl-phenylether und Polyoxyethylen(12)nonylphenylether.

5. Lyse-, Binde- und/oder Waschreagenz nach einem der vorherigen Ansprüche, **dadurch gekennzeichnet, dass** das Lyse-, Binde- und/oder Waschreagenz nicht-ionisches Tensid im Bereich von ≥ 9 % Gewicht/Volumen bis ≤ 40 % Gewicht/Volumen, vorzugsweise im Bereich von ≥ 10 % Gewicht/Volumen bis ≤ 30 % Gewicht/Volumen, bevorzugt im Bereich von ≥ 15 % Gewicht/Volumen bis ≤ 20 % Gewicht/Volumen, bezogen auf das Gesamtvolumen des Reagenzes, umfasst.

6. Lyse-, Binde- und/oder Waschreagenz nach einem der vorherigen Ansprüche, **dadurch gekennzeichnet, dass** die chaotrope Verbindung ein Natrium- oder Guanidiniumsalz ist, vorzugsweise ausgewählt aus der Gruppe umfassend Natriumiodid, Natriumperchlorat, Guanidiniumhydrochlorid, Guanidiniumthiocyanat, Guanidiniumisothiocyanat und/oder eine Mischung zweier oder mehrerer Salze davon.

7. Lyse-, Binde- und/oder Waschreagenz nach einem der vorherigen Ansprüche, **dadurch gekennzeichnet, dass** das Bindereagenz ein verzweigtes oder unverzweigtes Alkanol umfasst, bevorzugt einen verzweigten oder unverzweigten Alkohol mit einem bis fünf Kohlenstoffatomen, vorzugsweise ausgewählt aus der Gruppe umfassend Methanol, Ethanol, Isopropanol, n-Propanol, n-Butanol, verzweigtes oder unverzeigtes Butanol oder Pentanol und/oder Mischungen davon.

8. Verwendung eines Lyse-, Binde- und/oder Waschreagenzes nach einem der vorherigen Ansprüche zur Isolierung und/oder Reinigung von Nukleinsäuren.

9. Verfahren zur Isolierung und/oder Reinigung von Nukleinsäuren aus einer Nukleinsäuren enthaltenden biologischen Probe, umfassend folgende Verfahrensschritte:
a) Lysieren der biologischen Probe,
b) Immobilisieren der freigesetzten Nukleinsäure(n) an einer Matrix auf Basis einer oder mehrerer Siliciumoxidverbindung(en) in Gegenwart einer chaotropen Verbindung und/oder eines verzweigten oder unverzweigten Alkanols,
c) optional Waschen der auf der Matrix immobilisierten Nukleinsäure(n),
d) optional Abtrennen der gebundenen Nukleinsäure,
wobei man das Lysieren und/oder Immobilisieren in Gegenwart einer Lyse- und/oder Bindezusammensetzung durchführt, umfassend:
- wenigstens eine chaotrope Verbindung, - wenigstens eine Pufferverbindung, und
- wenigstens ein polyoxyethylen-basiertes nicht-ionisches Tensid ausgewählt aus der Gruppe bestehend aus Polyoxyethylennonylphenylether, Polyoxyethylen(5)isooctylphenylether und Polyoxyethylen(12)isooctylphenylether im Bereich von ≥ 0,1 % Gewicht/Volumen bis ≤ 50 % Gewicht/Volumen, bezogen auf das Gesamtvolumen der Zusammensetzung.

10. Verfahren nach Anspruch 9, **dadurch gekennzeichnet, dass** das polyoxyethylen-basierte nicht-ionische Tensid einen Polyoxyethylen-Bestandteil umfasst, der 2 bis 150 (CH₂CH₂O)-Einheiten enthält.

11. Verfahren nach Anspruch 9 oder 10, **dadurch gekennzeichnet, dass** der Polyoxyethylennonylphenylether ausgewählt ist aus der Gruppe bestehend aus Polyoxyethylen(2)nonylphenylether, Polyoxyethylen(5)nonylphenylether, Polyoxyethylen(9)nonylphenylether, Polyoxyethylen(12)nonylphenylether und Polyoxyethylen(100)nonylphenylether, vorzugsweise ausgewählt aus der Gruppe bestehend aus Polyoxyethylen(5)nonylphenylether, Polyoxyethylen(9)nonylphenylether und Polyoxyethylen(12)nonylphenylether.

12. Verfahren nach einem der vorherigen Ansprüche, **dadurch gekennzeichnet, dass** die Lyse- und/oder Bindezusammensetzung nicht-ionisches Tensid im Bereich von ≥ 0,2 % Gewicht/Volumen bis ≤ 30 % Gewicht/Volumen, vorzugsweise im Bereich von ≥ 3 % Gewicht/Volumen bis ≤ 10 % Gewicht/Volumen, bevorzugt im Bereich von ≥ 3,2 % Gewicht/Volumen bis ≤ 8 % Gewicht/Volumen, bezogen auf das Gesamtvolumen der Zusammensetzung, umfasst.

13. Kit zur Isolierung und/oder Reinigung von Nukleinsäuren aus einer Nukleinsäuren enthaltenden biologischen Probe, umfassend ein Lyse-, Binde- und/oder Waschreagenz nach einem der Ansprüche 1 bis 7.

14. Verwendung von polyoxyethylen-basierten nicht-ionischen Tensiden ausgewählt aus der Gruppe bestehend aus Polyoxyethylennonylphenylether, Polyoxyethylen(5)isooctylphenylether und Polyoxyethylen(12)isooctylphenylether, zur Herstellung lagerstabiler Binde-, Lyse- und/oder Waschreagenzien gemäß einem oder mehreren der Ansprüche 1 bis 7.

## Claims

1. Lysis, binding and/or wash reagent comprising:
- at least one chaotropic compound,
- at least one buffer compound, preferably selected from the group comprising tris(hydroxymethyl)aminomethane, N-(tri(hydroxymethyl)methyl)glycine, N,N-bis(2-hydroxyethyl)glycine, 3-(N-morpholino)propanesulphonic acid, N-(2-hydroxyethyl)piperazine-N'-(2-ethanesulphonic acid), piperazine-1,4-bis(2-ethanesulphonic acid), N-cyclohexyl-2-aminoethanesulphonic acid, 2-(N-morpholino)ethanesulphonic acid and/or phosphate buffer, and
- at least one polyoxyethylene-based non-ionic surfactant selected from the group consisting of polyoxyethylene nonylphenyl ether, polyoxyethylene (5) isooctylphenyl ether and polyoxyethylene (12) isooctylphenyl ether in a range from ≥ 8% weight/volume to ≤ 50% weight/volume, based on the total volume of the reagent.

2. Lysis, binding and/or wash reagent according to claim 1, **characterized in that** the polyoxyethylene-based non-ionic surfactant comprises a polyoxyethylene component containing from 2 to 150 (CH₂CH₂O) units.

3. Lysis, binding and/or wash reagent according to claim 1 or 2, **characterized in that** the polyoxyethylene nonylphenyl ether is selected from the group consisting of polyoxyethylene (2) nonylphenyl ether, polyoxyethylene (5) nonylphenyl ether, polyoxyethylene (9) nonylphenyl ether, polyoxyethylene (12) nonylphenyl ether and polyoxyethylene (100) nonylphenyl ether.

4. Lysis, binding and/or wash reagent according to any one of the preceding claims, **characterized in that** the polyoxyethylene nonylphenyl ether is selected from the group consisting of polyoxyethylene (5) nonylphenyl ether, polyoxyethylene (9) nonylphenyl ether and polyoxyethylene (12) nonylphenyl ether.

5. Lysis, binding and/or wash reagent according to any one of the preceding claims, **characterized in that** the lysis, binding and/or wash reagent comprises the non-ionic surfactant in the range from ≥ 9% weight/volume to ≤ 40% weight/volume, preferably in the range from ≥ 10% weight/volume to ≤ 30% weight/volume, preferentially in the range from ≥ 15% weight/volume to ≤ 20% weight/volume, based on the total volume of the reagent.

6. Lysis, binding and/or wash reagent according to any one of the preceding claims, **characterized in that** the chaotropic compound is a sodium salt or a guanidinium salt, preferably selected from the group comprising sodium iodide, sodium perchlorate, guanidinium hydrochloride, guanidinium thiocyanate, guanidinium isothiocyanate, and/or a mixture of two or more salts thereof.

7. Lysis, binding and/or wash reagent according to any one of the preceding claims, **characterized in that** the binding reagent comprises a branched or unbranched alkanol, preferably a branched or unbranched alcohol having from one to five carbon atoms, preferably selected from the group comprising methanol, ethanol, isopropanol, n-propanol, n-butanol, branched or unbranched butanol or pentanol, and/or mixtures thereof.

8. Use of a lysis, binding and/or wash reagent according to any one of the preceding claims for isolating and/or purifying nucleic acids.

9. Method for isolating and/or purifying nucleic acids from a nucleic acids containing biological sample, comprising the following method steps:
a) lysing the biological sample,
b) immobilizing the released nucleic acid(s) on a matrix that is based on one or more silicon oxide compound(s) in the presence of a chaotropic compound and/or a branched or unbranched alkanol,
c) optionally washing the nucleic acid(s) immobilized on the matrix,
d) optionally removing the bound nucleic acid(s),
wherein lysing and/or immobilizing is carried out in the presence of a lysis and/or binding composition comprising:
- at least one chaotropic compound,
- at least one buffer compound, and
- at least one polyoxyethylene-based non-ionic surfactant selected from the group consisting of polyoxyethylene nonylphenyl ether, polyoxyethylene (5) isooctylphenyl ether and polyoxyethylene (12) isooctylphenyl ether in the range from ≥ 0.1% weight/volume to ≤ 50% weight/volume, based on the total volume of the composition.

10. Method according to claim 9, **characterized in that** the polyoxyethylene-based non-ionic surfactant comprises a polyoxyethylene component containing from 2 to 150 (CH₂CH₂O) units.

11. Method according to claim 9 or 10, **characterized in that** the polyoxyethylene nonylphenyl ether is selected from the group consisting of polyoxyethylene (2) nonylphenyl ether, polyoxyethylene (5) nonylphenyl ether, polyoxyethylene (9) nonylphenyl ether, polyoxyethylene (12) nonylphenyl ether and polyoxyethylene (100) nonylphenyl ether, preferably selected from the group consisting of polyoxyethylene (5) nonylphenyl ether, polyoxyethylene (9) nonylphenyl ether and polyoxyethylene (12) nonylphenyl ether.

12. Method according to any one of the preceding claims, **characterized in that** the lysis and/or binding composition comprises the non-ionic surfactant in the range from ≥ 0.2% weight/volume to ≤ 30% weight/volume, preferably in the range from ≥ 3% weight/volume to ≤ 10% weight/volume, preferentially in the range from ≥ 3.2% weight/volume to ≤ 8% weight/volume, based on the total volume of the composition.

13. Kit for isolating and/or purifying nucleic acids from a nucleic acid-containing biological sample, comprising a lysis, binding and/or wash reagent according to any one of claims 1 to 7.

14. Use of polyoxyethylene-based non-ionic surfactants, selected from the group consisting of polyoxyethylene nonylphenyl ether, polyoxyethylene (5) isooctylphenyl ether and polyoxyethylene (12) isooctylphenyl ether, for preparing storage-stable binding, lysis and/or wash reagents according to one or more of claims 1 to 7.

## Revendications

1. Réactif de lyse, fixation et/ou lavage comprenant :
- au moins un composé chaotropique,
- au moins un composé tampon sélectionné de préférence parmi le groupe comprenant le tris(hydroxyméthyl)aminométhane, la N-(tri(hydroxyméthyl)méthyl)glycine, la N,N-bis(2-hydroxyéthyl)-glycine, l'acide 3-(N-morpholino)propanesulfonique, l'acide N-(2-hydroxyéthyl)pipérazine-N'-(2-éthanesulfonique), l'acide pipérazine-1,4-bis(2-éthanesulfonique), l'acide N-cyclohexyl-2-aminoéthanesulfonique, l'acide 2-(N-morpholino)-éthanesulfonique et/ou un tampon de phosphate, et
- au moins un tensioactif non ionique à base de polyoxyéthylène sélectionné parmi le groupe constitué de l'éther polyoxyéthylènenonylphénylique, l'éther polyoxyéthylène(5)isooctylphénylique et l'éther polyoxyéthylène(12)isooctylphénylique dans la plage de ≥ 8 % en poids/volume à ≤ 50 % en poids/volume, par rapport au volume total du réactif.

2. Réactif de lyse, fixation et/ou lavage selon la revendication 1, **caractérisé en ce que** le tensioactif non ionique à base de polyoxyéthylène comprend un constituant de polyoxyéthylène qui contient 2 à 150 unités (CH₂CH₂O).

3. Réactif de lyse, fixation et/ou lavage selon la revendication 1 ou 2, **caractérisé en ce que** l'éther polyoxyéthylènenonylphénylique est sélectionné parmi le groupe constitué de l'éther polyoxyéthylène(2)nonylphénylique, l'éther polyoxyéthylène(5)nonylphénylique, l'éther polyoxyéthylène(9)nonylphénylique, l'éther polyoxyéthylène(12)nonylphénylique et l'éther polyoxyéthylène(100)nonylphénylique.

4. Réactif de lyse, fixation et/ou lavage selon une des revendications précédentes, **caractérisé en ce que** l'éther polyoxyéthylènenonylphénylique est sélectionné parmi le groupe constitué de l'éther polyoxyéthylène(5)nonylphénylique, l'éther polyoxyéthylène(9)nonylphénylique et l'éther polyoxyéthylène(12)nonylphénylique.

5. Réactif de lyse, fixation et/ou lavage selon une des revendications précédentes, **caractérisé en ce que** le réactif de lyse, fixation et/ou lavage comprend un tensioactif non ionique dans la plage de ≥ 9 % en poids/volume à ≤ 40 % en poids/volume, de préférence dans la plage de ≥ 10 % en poids/volume à ≤ 30 % en poids/volume, de manière préférée dans la plage de ≥ 15 % en poids/volume à ≤ 20 % en poids/volume, par rapport au volume total du réactif.

6. Réactif de lyse, fixation et/ou lavage selon une des revendications précédentes, **caractérisé en ce que** le composé chaotropique est un sel de sodium ou guanidinium, sélectionné de préférence parmi le groupe comprenant l'iodure de sodium, le perchlorate de sodium, le chlorhydrate de guanidinium, le thiocyanate de guanidinium, l'isothiocyanate de guanidinium et/ou un mélange de deux ou plusieurs sels de ceux-ci.

7. Réactif de lyse, fixation et/ou lavage selon une des revendications précédentes, **caractérisé en ce que** le réactif de fixation comprend un alcanol ramifié ou non ramifié, de manière préférée un alcool ramifié ou non ramifié avec un à cinq atomes de carbone, sélectionné de préférence parmi le groupe comprenant le méthanol, l'éthanol, l'isopropanol, le n-propanol, le n-butanol, le butanol ou le pentanol ramifié ou non ramifié et/ou des mélanges de ceux-ci.

8. Utilisation d'un réactif de lyse, fixation et/ou lavage selon une des revendications précédentes pour l'isolation et/ou la purification d'acides nucléiques.

9. Procédé d'isolation et/ou de purification d'acides nucléiques à partir d'un échantillon biologique contenant des acides nucléiques, comprenant les étapes de procédé suivantes :
a) lyse de l'échantillon biologique,
b) immobilisation du/des acide(s) nucléique(s) libéré(s) sur une matrice à base d'un ou plusieurs composé(s) d'oxyde de silicium en présence d'un composé chaotropique et/ou d'un alcanol ramifié ou non ramifié,
c) lavage optionnel du/des acide(s) nucléique(s) immobilisé(s) sur la matrice,
d) séparation optionnelle de l'acide nucléique fixé,
dans lequel on réalise la lyse et/ou l'immobilisation en présence d'une composition de lyse et/ou de fixation, comprenant :
- au moins un composé chaotropique, - au moins un composé tampon, et
- au moins un tensioactif non ionique à base de polyoxyéthylène sélectionné parmi le groupe constitué de l'éther polyoxyéthylènenonylphénylique, l'éther polyoxyéthylène(5)isooctylphénylique et l'éther polyoxyéthylène(12)isooctylphénylique dans la plage de ≥ 0,1 % en poids/volume à ≤ 50 % en poids/volume, par rapport au volume total de la composition.

10. Procédé selon la revendication 9, **caractérisé en ce que** le tensioactif non ionique à base de polyoxyéthylène comprend un constituant de polyoxyéthylène qui contient 2 à 150 unités (CH₂CH₂O).

11. Procédé selon la revendication 9 ou 10, **caractérisé en ce que** l'éther polyoxyéthylènenonylphénylique est sélectionné parmi le groupe constitué de l'éther polyoxyéthylène(2)nonylphénylique, l'éther polyoxyéthylène(5)nonylphénylique, l'éther polyoxyéthylène(9)nonylphénylique, l'éther polyoxyéthylène(12)nonylphénylique et l'éther polyoxyéthylène(100)nonylphénylique, sélectionné de préférence parmi le groupe constitué de l'éther polyoxyéthylène(5)nonylphénylique, l'éther polyoxyéthylène(9)nonylphénylique et l'éther polyoxyéthylène(12)nonylphénylique.

12. Procédé selon une des revendications précédentes, **caractérisé en ce que** la composition de lyse et/ou de fixation comprend un tensioactif non ionique dans la plage de ≥ 0,2 % en poids/volume à ≤ 30 % en poids/volume, de préférence dans la plage de ≥ 3 % en poids/volume à ≤ 10 % en poids/volume, de manière préférée dans la plage de ≥ 3,2 % en poids/volume à ≤ 8 % en poids/volume, par rapport au volume total de la composition.

13. Ensemble d'isolation et/ou de purification d'acides nucléiques à partir d'un échantillon biologique contenant des acides nucléiques, comprenant un réactif de lyse, fixation et/ou lavage selon une des revendications 1 à 7.

14. Utilisation de tensioactifs non ioniques à base de polyoxyéthylène sélectionnés parmi le groupe constitué de l'éther polyoxyéthylènenonylphénylique, l'éther polyoxyéthylène(5)isooctylphénylique et l'éther polyoxyéthylène(12)isooctylphénylique, pour la fabrication de réactifs de fixation, lyse et/ou lavage stables au stockage selon une ou plusieurs des revendications 1 à 7.
